# EUROPÄISCHE PATENTSCHRIFT

(11) **EP 1 707 253 B1**
(45) Veröffentlichungstag und Bekanntmachung des Hinweises auf die Patenterteilung: **20.08.2008**
(21) Anmeldenummer: 06004551.5
(22) Anmeldetag: 07.03.2006
(51) Int. Cl.: B01D 59/44, C01B 3/10

(54) **Verfahren und Vorrichtung zur Bestimmung von Isotopenverhältnissen leichter Elemente in einem Analyseablauf**
Method and device for determination of isotope ratios of light elements in one analytical run
Procédé et dispositif destinés à la détermination de rapports isotopiques d'éléments légers en une étape

(30) Priorität: 29.03.2005 DE 102005014705
(43) Veröffentlichungstag der Anmeldung: 04.10.2006
(73) Patentinhaber: Elementar Analysensysteme GmbH, 63452 Hanau (DE); Isolab GmbH, 30966 Hemmingen (DE)
(72) Erfinder: Sieper, Hans-Peter, Dr., 63571 Gelnhausen (DE); Kupka, Hans-Joachim, 63543 Neuberg (DE); Schmidt, Hanns-Ludwig, Prof. Dr., 84036 Landshut (DE); Rossmann, Andreas, Dr., 85301 Schweitenkirchen 14 (DE)
(74) Vertreter: Grimm, Ekkehard

(56) Entgegenhaltungen:
- SIEPER H-P, WINKLER R: "Klasse mit Masse" LAB, [Online] Februar 2004 (2004-02), Seite 16,18, XP002387625 Gefunden im Internet: URL:http://dbindustrie.work.svhfi.de/AI/re sources/55c03750c73.pdf> [gefunden am 2006-06-28]
- MIDWOOD ANDREW J ET AL: "Recent developments in the analysis of light isotopes by continuous flow isotope ratio mass spectrometry" ANALYTICAL COMMUNICATIONS, Bd. 36, Nr. 8, August 1999 (1999-08), Seiten 291-294, XP002387626 ISSN: 1359-7337
- HUBER C ET AL: "Fast high-precision on-line determination of hydrogen isotope ratios of water or ice by continuous-flow isotope ratio mass spectrometry." RAPID COMMUNICATIONS IN MASS SPECTROMETRY, Bd. 17, Nr. 12, 2003, Seiten 1319-1325, XP002387627 ISSN: 0951-4198
- SHOUAKAR-STASH O ET AL: "On-line D/H analysis for water, natural gas, and organic solvents by manganese reduction" ANALYTICAL CHEMISTRY 01 JUN 2000 UNITED STATES, Bd. 72, Nr. 11, 1. Juni 2000 (2000-06-01), Seiten 2664-2666, XP002387628 ISSN: 0003-2700
- ANONYMOUS: "vario EL III: The ultimate standard CHNOS analysis" INTERNET ARTICLE, [Online] Oktober 2003 (2003-10), Seiten 1-8, XP002387629 Gefunden im Internet: URL:http://www.houm.no/pdf/Elementar_vario _EL_III__E_.pdf> [gefunden am 2006-06-28]
- JASPER J P ET AL: "Stable isotopic characterization of active pharmaceutical ingredients" JOURNAL OF PHARMACEUTICAL AND BIOMEDICAL ANALYSIS, Bd. 35, Nr. 1, 1. April 2004 (2004-04-01), Seiten 21-30, XP002387630 ISSN: 0731-7085

## Beschreibung

Die Erfindung betrifft ein Verfahren gemäß dem Oberbegriff des Anspruchs 1. Weiterhin betrifft die Erfindung Vorrichtungen gemäß den Oberbegriffen der Ansprüche 14 und 15.

Mit Hilfe der Isotopenverhältnis-Massenspektrometrie (IRMS - "Isotope Ratio Mass Spectrometry") lassen sich die Isotopenverhältnisse biologisch wichtiger Elemente, wie Stickstoff, Kohlenstoff und Schwefel, bestimmen. Eine wesentliche Anwendung ist der Nachweis der (geographischen) Herkunft des biologischen Materials und der Naturbelassenheit (Authentizität) von Lebensmitteln, da die natürlichen Isotopenverhältnisses der Elemente, aus denen sie aufgebaut sind, durch die (bio)chemischen, geographischen und botanischen Gegebenheiten am Entstehungsort festgelegt oder beeinflusst werden. Eine ausreichende Information über Herkunft und Behandlung solcher Proben ist dabei jedoch nur durch eine Multielement-Isotopenanalyse möglich, da insbesondere für die Erkennung der geographischen Herkunft die Isotopenverhältnisse der Elemente Wasserstoff und Sauerstoff typisch sind; für Hinweise auf andere biochemische Parameter, wie Pflanzentyp, Dünger und Bodenart, die Isotopenverhältnisse der Elemente Kohlenstoff, Stickstoff und Schwefel.

Oft wird zwar eine geographische Zuordnung allein auf der Basis des (einfach zu bestimmenden) δ¹⁸O-Wertes des Wassers der Lebensmittel versucht, doch unterliegt dieser Wert häufig sekundären Veränderungen durch Verarbeitung, Transport und Lagerung. Deshalb ist auch die Bestimmung der Isotopenverhältnisse der anderen Elemente unabdingbar; hierfür müssen meistens mehrere unabhängige Analysen durchgeführt werden.

Das Dokument Sieper H.-P., Winkler R. "Klasse mit Masse" @LAB, [Online], Februar 2004, Seite 16, 18, das einen Stand der Technik entsprechend den Oberbegriffen der Ansprüche 1, 14 und 15 darstellt, beschreibt auf Seite 18, linke Spalte, die "Purge- und Trap"-Gastrenntechnik, die die Simultananalyse von CNS-Isotopen auch bei stark auseinander liegenden Elementkonzentrationen erlaubt. Die hierzu eingesetzten Systeme basieren auf der Kopplung von Elementaranalysatoren für die CNS- und O-Isotopenanalytik. Es wird auch die Möglichkeit einer Kopplung mit anderen IRMS-Systemen noch höherer Leistung bzw. solchen für die Wasserstoff-Isotopenbestimmung angesprochen. Bei den in diesem Dokument angegebenen Systemen erfolgt demnach die Wasserstoff-Isotopenbestimmung in einer zusätzlichen Einrichtung zusätzlich zu der Anordnung für die Simultananalyse von C, N und S. Es handelt sich folglich bei dem System für die Wasserstoff-Isotopenbestimmung um ein System, das zwei Analysenabläufe für die CNSH-Isotopenbestimmung erfordert, nämlich einen Analysenablauf für die CNS-Isotopenbestimmung und einen weiteren Analysenablauf für die H-Isotopenbestimmung.

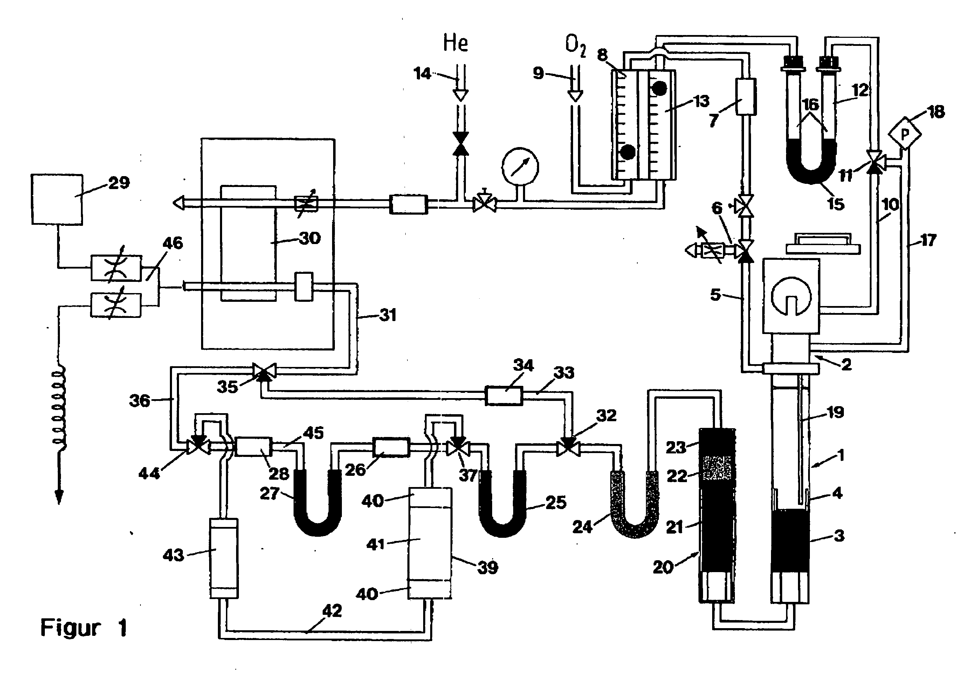

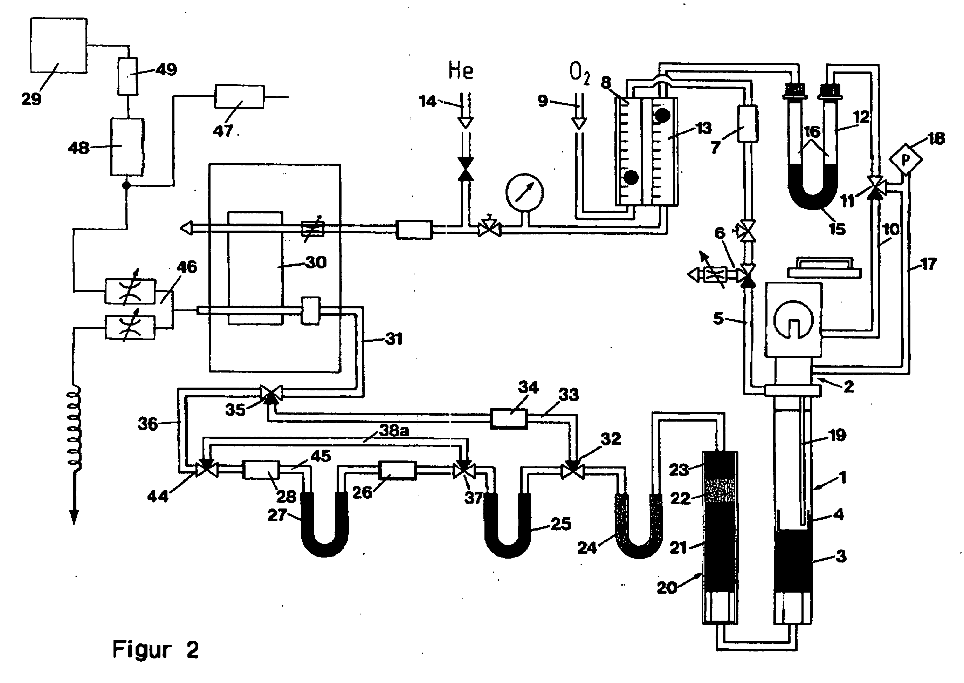

Das Dokument MIDWOOD. ET AL., "Recent developments in the analysis of light isotopes by continuous flow isotope ratio mass spectrometry" ANAL COMMUN, 36(8), 1999, Seiten 291-294, beschreibt die Messung der Isotopen von Stickstoff und Kohlenstoff in festen oder flüssigen Proben sowie von Wasserstoff und Schwefel in separaten Messdurchläufen. Die Anordnung, wie sie in der Figur 1 gezeigt ist, dient nur zur Bestimmung der Isotopen von N und C, und, wie anhand der schematischen Vorrichtung dieser Figur zu sehen ist, wird H₂O entfernt.

Das Dokument HUBER C ET AL: "Fast high-precision on-line determination of hydrogen isotope ratios of water or ice by continuous-flow isotope ratio mass spectrometry". RAPID COMMUN MASS SPEC, 17(12), 2003, Seiten 1319-1325, beschreibt ausschließlich die Online-Bestimmung der Wasserstoffisotopenverhältnisse von Wasser oder Eis mittels der "continuous-flow isotope ratio mass spectrometry". Es wird in diesem Dokument Magnesium in Bezug auf Wasser-Wasserstoff-Umwandlungsverfahren erwähnt; jedoch bezieht sich eine Arbeit, in Bezug auf die Magnesium erwähnt wird, auf platiniertes Magnesium.

Das Dokument SHOUAKAR-STASH O ET AL: "On-line D/H analysis for water, natural gas, and organic solvents by manganese reduction" ANAL CHEM, 72(11), 2000, Seiten 2664-2666, beschreibt ausschließlich die Wasserstoff-Isotopenmessung. Soweit Magnesium in D4 als Reduktionsmittel erwähnt ist, wird auch hier reduziertes Magnesium angebeben.

Der vorliegenden Erfindung liegt nun die Aufgabe zugrunde, ein Verfahren für die simultane Bestimmung der Isotopenverhältnisse der Elemente Kohlenstoff (C), Stickstoff (N) und Schwefel (S) und eine entsprechende Vorrichtung derart auszubilden, dass die geographische Herkunft, die Authentizität und die Naturbelassenheit von Lebensmitteln sicher bestimmt werden können, indem erfindungsgemäß die Isotopenverhältnisse von Wasserstoff (H) zusätzlich parallel gemessen werden können. Optional soll auch eine Sauerstoff-(O)-Isotopenanalyse möglich sein.

Gelöst wird diese Aufgabe durch ein Verfahren, das die Merkmale gemäß Anspruch 1, aufweist, und durch Vorrichtungen, die zum einen die Merkmale des Anspruchs 14 und zum anderen die Merkmale des Anspruchs 15 aufweisen.

Bisher erfolgt die IRMS-Analytik in wenigen großen Speziallabors, da die verfügbaren Geräte hohe Anforderungen an Betriebs- und Laborbedingungen stellen und sehr kostenaufwändig sind. Zudem weisen einsetzbare Verfahren zur Isotopenverhältnisanalyse von organisch gebundenem Wasserstoff und Sauerstoff Schwächen auf bzw. sind aufwändig, da derzeit Wasserstoff-Isotopenverhältnisse nicht simultan zu denen anderer Elemente bestimmt werden können. Derzeitige Geräte erlauben auch nur die Analyse von wenigen Milligramm Probenmaterial, was für die Lebensmittelanalytik nachteilig ist, da es sich häufig um heterogenes Probenmaterial handelt, in dem die genannten Elemente in sehr verschiedenen Verhältnissen vorkommen können.

Derzeit auf dem Markt erhältliche Geräte zur Isotopenverhältnisanalyse sind im Wesentlichen aus zwei Komponenten aufgebaut, dem vorgeschalteten Elementaranalysator und dem nachgeschalteten Massenspektrometer. Die Massenspektrometer-Hersteller bieten meist komplette Systeme an, deren Elementaranalysator aber oft nicht den Ansprüchen in der Lebensmittelanalytik genügt (große Proben, heterogene Zusammensetzung der Proben, extreme Elementverhältnisse).

Im Gegensatz dazu ist es mit dem erfindungsgemäßen Verfahren und der erfindungsgemäßen Vorrichtung möglich, eine simultane, schnelle und kostengünstige Analyse mehrerer Parameter bezüglich Herkunft bzw. Verfälschung von Lebensmitteln durchzuführen. Bezüglich ihrer Herkunft falsch deklarierte bzw. mit unzulässigen Zusätzen verfälschte Lebensmittel können auf diese Weise schnell aus dem Verkehr gezogen werden und würden gar nicht erst in den Handel und zum Verbraucher gelangen.

Im Elementaranalysator erfolgt zunächst der vollständige Probenaufschluss durch eine kontrollierte quantitative Verbrennung der Probe bei 950° - 1200°C. Die daraus resultierenden Verbrennungsgase N₂, CO₂, SO₂ und H₂O-Dampf werden mittels "Purge-and-trap"-Technik getrennt und in spezifisch wirkenden Adsorptionssäulen gesammelt. Die Gase N₂, nach Aufheizen der Adsorptionssäulen auf spezifische Temperaturen auch CO₂ und SO₂, gelangen dann in ein Isotopenverhältnis-Massenspektrometer, in dem die Messung der Isotopenverhältnisse ¹⁴N/¹⁵N, ¹²C/¹³C, ³²S/³⁴S erfolgt. Das Isotopenverhältnis ¹H/²H musste hierfür in einem besonderen Verfahren (pyrolytischer Aufschluss der Probe) bestimmt werden, da H₂O zur massenspektrometrischen Isotopenverhältnisanalyse ungeeignet ist.

Aufgrund des erfindungsgemäßen Verfahrens und der erfindungsgemäßen Vorrichtung muss die Analyse der Wasserstoff-Isotope nicht getrennt von der Analyse der anderen Elemente erfolgen, da der H₂O-Dampf über ein sich in einem Ofen befindendes Reduktionsmittel geführt wird, um ihn zu H₂-Gas zu reduzieren, oder ihn mit H₂ bekannten Isotopengehaltes zu äquilibrieren, wodurch Aufwand und Kosten gegenüber bisherigen Verfahren und Vorrichtungen herabgesetzt werden.

Gemäß der Erfindung sind die routinemäßige Bestimmung von δ¹⁸O-Werten sowie die simultane Bestimmung der δ¹³C-, δ²H-, δ ¹⁵N- und δ³⁴S-Werte einer Probe möglich.

Die Isotopenanalyse von wenig Schwefel neben viel Kohlenstoff, was zum Beispiel typisch für Lebensmittel ist, ist mit dem erfindungsgemäßen Verfahren und der entsprechenden Vorrichtung möglich, indem große Probenmengen bis zu 40 mg organischen Materials analysiert werden können.

Die simultane Messung von mehreren Elementen, insbesondere auch unter der gleichzeitigen Bestimmung von Wasserstoff, wird möglich, da anstelle einer gaschromatografischen Gastrennung die "Purge-and-trap"-Technik verwendet wird.

Durch einen einfachen und kompakten Aufbau der Vorrichtung können die Kosten der Vorrichtung wesentlich im Vergleich zu bekannten Geräten und Systemen verringert werden. Der Zeitbedarf für die Analyse einer Probe auf CHNS-Isotopenverhältnisse wird so um mindestens die Hälfte verringert, so dass der Probendurchsatz des Gerätes, im Vergleich zu bekannten Geräten, entsprechend erhöht werden kann.

Zur Reduktion des Wasserdampfes zu Wasserstoff wird bevorzugt Mangan als Katalysator eingesetzt, wobei die Reaktionstemperatur 850° bis 1000°C beträgt.

Alternativ zu Mangan kann auch Magnesium eingesetzt werden, wobei die Reaktionstemperatur 400° bis 600°C beträgt. Die Anwendung anderer Metalle oder deren Kombinationen sind unter Beachtung der speziellen Einsatzbedingungen ebenfalls möglich.

Zur Umgehung der Hochtemperatur-Reduktion kann für die Gewinnung des Messgases H₂ alternativ eine Isotopenaustauschmethode verwendet werden. Hierbei wird der von der H₂O-Adsorptionssäule desorbierte Wasserdampf in Gegenwart eines Platinkatalysators mit Wasserstoff eines bekannten ¹H/²H-Verhältnisses zusammengebracht. Wasserstoff und Wasserdampf äquilibrieren dabei ihren Isotopengehalt, so dass der des Wasserstoffs ein Maß für den des Wasserdampfes darstellt. Diese Methode setzt allerdings voraus, dass die Wasserdampf- und die Wasserstoffmenge bekannt sind. Zur Ermittlung der ersteren wird die entsprechende Messeinrichtung des Elementaranalystors (WLD) verwendet, die Wasserstoffmenge wird über Druck und Durchflussrate eingestellt.

Weitere vorteilhafte Ausgestaltungen des erfindungsgemäßen Verfahrens und der erfindungsgemäßen Vorrichtung ergeben sich aus den abhängigen Ansprüchen.

Eine beispielhafte Vorrichtung gemäß der Erfindung wird nachfolgend anhand der beigefügten Zeichnung erläutert.

Die Vorrichtung, wie sie in der beigefügten Figur zu sehen ist, umfasst einen Verbrennungsofen 1, der mit einem Probenzuführungskopf 2 verschlossen ist. Der Verbrennungsofen 1 ist rohrförmig aufgebaut. Im unteren Bereich des rohrförmigen Verbrennungsofens 1 ist ein Oxidationskatalysator 3, beispielsweise Wolfram-VI-oxid, eingefüllt, auf dem ein Tiegel 4 zur Aufnahme einer Probe, in flüssiger oder fester Form, aufgesetzt ist. Die Probenmenge kann bis zu 100 mg bzw. 40 mg organische Substanz betragen.

Der Probenzuführungskopf 2 weist eine Zuführungsleitung 5 für Sauerstoff (O₂) auf, die über ein Dosierventil 6, ein Trockenmittel 7 und eine Messeinrichtung 8 mit einer Sauerstoffversorgung 9 verbunden ist. Weiterhin ist mit dem Probenzuführungskopf 2 eine Helium-Trägergasstrom-Zuführleitung 10 verbunden, die über ein Dosierventil 11, ein U-förmiges Gasreinigungsohr 12, eine weitere Messeinrichtung 13 mit einer Heliumversorgung 14 verbunden ist. Das U-Rohr 12 ist in seinem unteren Bogenbereich mit einem Absorptionsmittel 15, beispielsweise NaOH und in seinen beiden Schenkeln mit einem Trokkenmittel 16, und an den Enden jeweils durch Quarzwolle verschlossen, gefüllt. Weiterhin ist der Probenzuführungskopf 2 mit einer Druckmessleitung 17 und einer Druckanzeige 18 verbunden

Um die in den Tiegel 4 eingefüllte Probe in einem Helium-Trägergasstrom unter Zusatz von Sauerstoff durch Verbrennung aufzuschließen, kann die erforderliche Menge an Sauerstoff unmittelbar durch eine Lanze 19 zugeführt werden, die sich mit ihrem Ende direkt in den Tiegel 4 hinein erstreckt.

Die während der Verbrennung entstehenden gasförmigen Verbrennungsprodukte werden über das untere Ende des rohrförmigen Verbrennungsofens 1 in ein Reduktionsrohr 20 von der Unterseite zur Oberseite hin geführt, das mit Kupfergranulat 21, anschließend mit Korundkugeln 22 und einem sich daran anschließenden Halogenabsorber 23, beispielsweise Ag-Wolle, gefüllt ist. In diesem Rohr erfolgen die Absorption des überschüssigen Sauerstoffs sowie die Reduktion von Stickoxiden zu N₂ und von SO₃ zu SO₂. An das Rohr 20 schließt sich ein U-förmiges Rohr mit einem Schwefeldioxid (SO₂)-Adsorber 24 an. In Strömungsrichtung gesehen schließt sich an diesen SO₂-Adsorber ein U-förmiges Rohr mit einem H₂O-Adsorber 25 an, an dessen ausgangsseitigen Schenkel sich, über ein Trockenmittel 26 geführt, ein U-förmiges Rohr mit einem Kohlendioxid-Adsorber 27 anschließt. Das U-förmige Rohr mit dem Kohlendioxid-Adsorber 27 ist ausgangsseitig über ein weiteres Trockenmittel 28 über die Messvorrichtung 30 des Elementaranalysators und über einen Gasteiler 46, der zwei Nadelventile aufweist, mit einer IRMS-Analysevorrichtung (IRMS - Isotope Ratio Mass Spectrometry) 29 verbunden.

Die Anordnung, wie sie vorstehend mit den angegebenen Komponenten beschrieben ist, arbeitet wie folgt. Die in den Tiegel 4 eingegebene Probe, fest oder flüssig, wird in einem Helium-Trägergasstrom unter Zusatz von Sauerstoff zu Wasserdampf, Kohlendioxid, Stickstoff und Schwefeldioxid verbrannt. Diese Verbindungen werden dann über das Rohr 20 geführt, wo Restsauerstoff absorbiert, Stickoxide zu N₂ und SO₃ zu SO₂ reduziert werden. Schwefeldioxid, Wasserdampf, Kohlendioxid und Stickstoff werden dann über Säule 24 geleitet, wo das SO₂ adsorbiert wird. In der folgenden Adsorptionssäule 25 wird der H₂O-Dampf adsorbiert. Der Gasstrom wird dann dem CO₂-Adsorber über das Trocknungsmittel 26, um Kontaminationsfeuchte zurückzuhalten, zugeführt, wo das Kohlendioxid adsorbiert wird. Stickstoff, der durch keinen der Adsorptionsfilter zurückgehalten wird, wird dann unmittelbar mit dem Trägergasstrom über die Leitung 31 der IRMS-Messeinrichtung 29 zur Erfassung zugeführt. Nach dem Ende der N₂-Messung wird die CO₂-Adsorptionssäule zur Desorption des CO₂ aufgeheizt, wobei nun das CO₂ ebenfalls über die Leitungen 45, 36 und 31 der Messeinrichtung zugeführt wird.

Ausgangsseitig des SO₂-Adsorbers befindet sich ein Zweiwegeventil 32, das zwischen einer Zuführung des Gasstroms zu dem H₂O-Adsorber 25 und einer Abzweigungsleitung 33 umschaltbar ist.

Zur SO₂-Desorption / SO₂-Messung wird das Zweiwegeventil 32 so umgestellt, dass der Trägergasstrom über ein Trockenmittel 34 und die Leitung 31 der IRMS-Messeinrichtung 29 direkt zugeführt werden kann, wozu über ein weiteres Zweiwegeventil 35 die Leitung 36, die mit dem CO₂-Adsorber 27 und dem Trockenmittel 28 verbunden ist, von der Zuführleitung 31 abgetrennt werden kann.

Unmittelbar hinter dem H₂O-Adsorber 25 ist, vor dem Trockenmittel 26 und dem CO₂-Adsorber, ein zusätzliches Zweiwegeventil 37 eingebaut, das so geschaltet werden kann, dass der Gasstrom zu dem CO₂-Adsorber unterbrochen wird und er stattdessen über eine Abzweigleitung 38 einem in einem Reduktionsofen, der nicht näher dargestellt ist, befindlichen Reduktionsrohr 39 zugeführt wird. Das Reduktionsrohr 39, das an seiner Ober- und Unterseite mit Quarzwolle 40 verschlossen ist, ist mit einem Granulat 41, entweder Mangangranulat oder Magnesiumgranulat, gefüllt. Nach Aufheizen des H₂O-Absorbers wird das im Gasstrom enthaltene H₂O dem Reduktionsrohr 39 zugeführt und durch Magnesium- oder Mangangranulat dann reduziert. Anschließend wird der Gasstrom über die Leitung 42 am unteren Ende des Reduktionsrohrs 39 und ein Trockenmittel 43 über ein Zweiwegeventil 44 der Leitung 36 und damit der IRMS-Messeinrichtung zugeführt. Über das Zweiwegeventil 44 wird zwischen der Leitung 42 und der Leitung 45 umgeschaltet.

Alternativ zur Reduktion kann das Wasser mit H₂-Gas einer Isotopenäquilibrierung unterzogen werden. Hierzu wird der Trägerstrom mit dem H₂O-Dampf anstelle zum Reduktionsrohr 39 vom Ventil 37 aus, wie in der Anordnung der Figur 1, über die Leitung 38a dem Wärmeleitfähigkeitsdetektor 30 zugeführt, wo die Menge des Wassers gemessen wird. Im Anschluss wird dem Gasstrom über die Einheit 47 H₂-Gas bekannten Isotopengehaltes beigemischt (ca. 1 ml/min). Das Gemisch passiert dann den Äquilibrierungsreaktor 48 (ein Edelstahlrohr von ca. 5 ml Inhalt bei 100°C, gefüllt mit ca. 100 "Hokko Beads" (1 % Pt auf einem Träger)) und anschließend eine Trocknungseinheit 49 zur Abtrennung des Wassers. Dann gelangt es in das Massenspektrometer 29 zur Isotopen-Verhältnismessung,

## Patentansprüche

1. Verfahren für die Bestimmung der Isotopenverhältnisse der Elemente Kohlenstoff (C), Stickstoff (N) und Schwefel (S) in einem Analyseablauf mit einem Elementaranalysator in Verbindung mit einem Massenspektrometer (EA-IRMS), wobei die Verbrennungsgase aus den Elementen C, und S von N₂ durch zumindest eine elementspezifische, thermisch gesteuerte Adsorptionssäule für SO₂ und dann durch eine elementspezifische, thermisch gesteuerte Adsorptionssäule für CO₂ getrennt und einer IRMS-Messung zugeführt werden,
**dadurch gekennzeichnet,**
**dass** zur Bestimmung der Isotopenverhältnisse von C, N, S und zusätzlich auch von H in einem Analyseablauf die Verbrennungsgase durch eine weitere Adsorptionssäule zur Abtrennung des Wasserdampfs, der aus der Verbrennung des Wasserstoffs der Probe entsteht, geführt werden, die zwischen der Adsorptionssäule für SO₂ und der Adsorptionssäule für CO₂ zwischengefügt ist, und dass zur Bestimmung des Isotopenverhältnisses von H
der Gasstrom von der weiteren Adsorptionssäule, die zur Abtrennung des Wasserdampfs dient, zu der CO₂-Adsorptionssäule unterbrochen wird und der Gasstrom entweder über einen Reduktionsofen geführt und der IRMS-Messung zugeführt wird,
oder mit H₂-Gas zur Isotopenäquilibrierung gebracht wird und der Wasserstoff der IRMS-Messung zugeführt wird.

2. Verfahren nach Anspruch 1, **dadurch gekennzeichnet, dass** ein sich im Reduktionsofen befindliches Reduktionsrohr mit Magnesiumspänen gefüllt ist.

3. Verfahren nach Anspruch 1, **dadurch gekennzeichnet, dass** ein sich im Reduktionsofen befindliches Reduktionsrohr mit Mangangranulat gefüllt ist.

4. Verfahren nach Anspruch 1, **dadurch gekennzeichnet, dass** ein sich im Reduktionsofen befindliches Reduktionsrohr mit Metallen oder Metallkombinationen geeigneter Elektronegativität gefüllt ist.

5. Verfahren nach Anspruch 1, **dadurch gekennzeichnet, dass** eine Pt-katatysierte Isotopenäquilibrierung bei 100 °C zwischen Wasserdampf und Wasserstoffgas bekannten Isotopengehaltes in einem Gefäß durchgeführt wird und das Wasser anschließend in einer Trocknungseinheit abgetrennt wird.

6. Verfahren nach Anspruch 5, **dadurch gekennzeichnet, dass** die Abtrennung des Wassers in einem Nafion^{®}-Rohr durchgeführt wird.

7. Verfahren nach einem der Ansprüche 1 bis 4, **dadurch gekennzeichnet, dass** die Verbrennungsprodukte ausgangsseitig des Reduktionsofen einer Trocknung zum Schutz vor Kontaminierung mit Fremdfeuchtigkeit unterworfen werden.

8. Verfahren nach Anspruch 7, **dadurch gekennzeichnet, dass** die Trocknung in einem mit Phosphorpentoxid gefüllten Trockenrohr erfolgt.

9. Verfahren nach Anspruch 1, **dadurch gekennzeichnet, dass** die H₂O und Wasserstoffmenge durch Druckmessung bzw mittels Wärmeleitfähigkeitsdetektor (WLD) bestimmt wird.

10. Verfahren nach Anspruch 1, **dadurch** das die Trehnsäulen eine ausreichende Menge an spezifischem Adsorptionmaterial für Probenmengen oberhalb von 10 mg entfalten.

11. Verfahren nach einem der Ansprüche 1 bis 10, **dadurch gekennzeichnet, dass** die Schnittstelle zwischen der Elementaranalyse und der Isotopenverhältnis-Massenspektrometer-Analytik gemäß dem "Continuous flow" Prinzip aufgebaut ist.

12. Verfahren nach einem der Ansprüche 1 bis 11, **dadurch gekennzeichnet, dass** für die Bestimmung der Isotopenverhältnisse von C, H, N und S Probeneinwaagen bis 100 mg eingesetzt werden.

13. Vorrichtung für die Bestimmung der Isotopenverhältnisse der Elemente Kohlenstoff (C), Stickstoff (N) und Schwefel (S) in einem Analyseablauf, die einen Elementaranalysator mit einem Verbrennungsofen (1) zur Verbrennung der Probe, einer nachfolgenden Säule (24), der die Verbrennungsgase zugeführt werden, die mit einem SO₂-Adsorptionsmittel gefüllt ist, und einer sich daran anschließenden Säule (27), die mit einem CO₂-Adsorptionsmittel gefüllt ist, aufweist, und wobei die Verbrennungsgase nach der mit dem CO₂-Adsorptionsmittel gefüllten Säule (27) über einen Wärmeleitfähigkeitsdetektor (30) einer IRMS-Messeinrichtung (29) zugeführt werden,
**dadurch gekennzeichnet,**
**dass** zur Bestimmung der Isotopenverhältnisse von C, N, S und zusätzlich auch von H in einem Analyseablauf eine weitere Adsorptionssäule (25) zur Abtrennung des Wasserdampfs vorgesehen ist, die zwischen der Adsorptionssäule (24) für SO₂ und der Adsorptionssäule (27) für CO₂ zwischengefügt ist, und
**dass** vor und hinter der Adsorptionssäule (27) für CO₂ jeweils ein Mehrwegeventil (37; 44) vorgesehen ist, die so umschaltbar sind, dass der Gasstrom durch das eine Mehrwegeventil (37) von der weiteren Adsorptionssäule (25), die zur Abtrennung des Wasserdampfs dient, zu der CO₂-Adsorptionssäule (27) unterbrochen werden kann und der Gasstrom von diesem Mehrwegeventil (37) über einen Reduktionsofen (39) und das andere Mehrwegeventil (44) der IRMS-Messung(29) zugeführt wird.

14. Vorrichtung für die Bestimmung der Isotopenverhältnisse der Elemente Kohlenstoff (C), Stickstoff (N) und Schwefel (S) in einem Analyseablauf, die einen Elementaranalysator mit einem Verbrennungsofen (1) zur Verbrennung der Probe, einer nachfolgenden Säule (24), der die Verbrennungsgase zugeführt werden, die mit einem SO₂-Adsorptionsmittel gefüllt ist, und einer sich daran anschließenden Säule (27), die mit einem CO₂-Adsorptionsmittel gefüllt ist, aufweist, und wobei die Verbrennungsgase nach der mit dem CO₂-Adsorptionsmittel gefüllten Säule (27) über einen Wärmeleitfähigkeitsdetektor (30) einer IRMS-Messeinrichtung (29) zugeführt werden,
**dadurch gekennzeichnet,**
**dass** zur Bestimmung der Isotopenverhältnisse von C, N, S und zusätzlich auch von H in einem Analyseablauf eine weitere Adsorptionssäule (25) zur Abtrennung des Wasserdampfs vorgesehen ist, die zwischen der Adsorptionssäule (24) für SO₂ und der Adsorptionssäule (27) für CO₂ zwischengefügt ist, und
**dass** vor und hinter der Adsorptionssäule (27) für CO₂ jeweils ein Mehrwegeventil (37; 44) vorgesehen ist, die so umschaltbar sind, dass der Gasstrom durch das eine Mehrwegeventil (37) von der weiteren Adsorptionssäule (25), die zur Abtrennung des Wasserdampfs dient, zu der CO₂-Adsorptionssäule (27) unterbrochen werden kann und der Gasstrom von dem Mehrwegeventil (37) über das andere Mehrwegventil (44) einer Äquilibrierungseinheit (48), die mit der IRMS-Messeinrichtung (29) verbunden ist, zusammen mit H₂-Gas bekannten Isotopengehaltes das von einer Einheit (47) bereitgestellt wird, zugeführt wird.

15. Vorrichtung nach Anspruch 14, **dadurch gekennzeichnet, dass** die Äquilibrierungseinheit (48) ein H-Austauschreaktor ist.

16. Vorrichtung nach Anspruch 13, **dadurch gekennzeichnet, dass** ein sich im Reduktionsofen befindliches Reduktionsrohr (39) mit Metallen oder Metallkombinationen geeigneter Elektronegativität gefüllt ist.

17. Vorrichtung nach Anspruch 16, **dadurch gekennzeichnet, dass** das Reduktionsrohr (39) im Reduktionsofen mit Magnesiumspänen gefüllt wird.

18. Vorrichtung nach Anspruch 16, **dadurch gekennzeichnet, dass** das Reduktionsrohr (39) im Reduktionsofen mit Mangangranulat gefüllt ist.

19. Vorrichtung nach Anspruch 14, **dadurch gekennzeichnet, dass** anschließend an die Äquilibrierungseinheit (48) eine Vorrichtung (49) zur Abtrennung des Wassers eingesetzt ist.

20. Vorrichtung nach Anspruch 13, **dadurch gekennzeichnet, dass** die Verbrennungsprodukte ausgangsseitig des Reduktionsrohrs (39) durch ein Trockenrohr (43) geführt werden.

21. Vorrichtung nach Anspruch 20, **dadurch gekennzeichnet, dass** das Trockenrohr (43) mit Phosphorpentoxid gefüllt ist.

22. Vorrichtung nach einem der Ansprüche 13 bis 21, **dadurch gekennzeichnet, dass** die Adsorptionssäulen (24, 25, 27) für Probenmengen oberhalb von 10 mg ausgelegt sind.

23. Vorrichtung nach einem der Ansprüche 13 bis 22, **dadurch gekennzeichnet, dass** für die Adsorption/Desorption entsprechende Trennsäulen eingesetzt sind.

24. Vorrichtung nach einem der Ansprüche 13 bis 23, **dadurch gekennzeichnet, dass** die Schnittstelle zwischen der Elementaranalyse und der Isotopen-Massenspektrometer-Analytik als "Continuous-flow" Prinzip aufgebaut ist.

## Claims

1. A method for determining the isotope ratios of the elements carbon (C), nitrogen (N) and sulfur (S) in one analytical run with an elemental analyzer in combination with a mass spectrometer (EA-IRMS), the combustion gases from the elements C and S being separated from N₂ by at least one element-specific, thermally controlled adsorption column for SO₂ and then by an element-specific, thermally controlled adsorption column for CO₂ and subjected to an IRMS measurement,
**characterized in**
**that** for the determination of the isotope ratios of C, N, S and additionally also of H in one analytical run the combustion gases are passed through a further adsorption column for separating the water vapor arising from the combustion of the hydrogen of the sample, which further adsorption column is interposed between the adsorption column for SO₂ and the adsorption column for CO₂, and that for the determination of the isotope ratio of H
the gas flow from the further adsorption column, which serves to separate the water vapor, to the CO₂ adsorption column is interrupted and the gas flow is either passed via a reduction furnace and subjected to the IRMS measurement
or is isotope equilibrated with H₂ gas and the hydrogen is subjected to the IRMS measurement.

2. The method according to claim 1, **characterized in that** a reduction tube positioned in the reduction furnace is filled with magnesium chips.

3. The method according to claim 1, **characterized in that** a reduction tube positioned in the reduction furnace is filled with manganese granulate.

4. The method according to claim 1, **characterized in that** a reduction tube positioned in the reduction furnace is filled with metals or metal combinations of suitable electronegativity.

5. The method according to claim 1, **characterized in that** a Pt-catalyzed isotope equilibration is carried out at 100°C between water vapor and hydrogen gas of known isotope content in a vessel and the water is then separated in a drying unit.

6. The method according to claim 5, **characterized in that** separation of the water is carried out in a Nafion^{®} tube.

7. The method according to any one of claims 1 to 4, **characterized in that** the combustion products at the output side of the reduction furnace are subjected to drying for protection from contamination with external moisture.

8. The method according to claim 7, **characterized in that** drying is carried out in a drying tube filled with phosphorus pentoxide.

9. The method according to claim 1, **characterized in that** the H₂O and hydrogen amount is determined by pressure measurement and by means of a thermal conductivity detector (TCD), respectively.

10. The method according to claim 1, **characterized in that** the separation columns contain an adequate amount of specific adsorption material for sample amounts above 10 mg.

11. The method according to any one of claims 1 to 10, **characterized in that** the interface between the elemental analysis and the isotope ratio mass spectrometer analysis is designed according to the "continuous flow" principle.

12. The method according to any one of claims 1 to 11, **characterized in that** weighed portions of up to 100 mg are used for determining the isotope ratios of C, H, N and S.

13. A device for determining the isotope ratios of the elements carbon (C), nitrogen (N) and sulfur (S) in one analytical run, said device comprising an elemental analyzer with a combustion furnace (1) for burning the sample, a successive column (24) fed with the combustion gases, which is filled with an SO₂ adsorbent, and a successive column (27) filled with a CO₂ adsorbent, and the combustion gases after the column (27) filled with the CO₂ adsorbent being passed via a thermal conductivity detector (30) to an IRMS measuring device (29),
**characterized in**
**that** for the determination of the isotope ratios of C, N, S and additionally also of H in one analytical run a further adsorption column (25) is provided for separating the water vapor, said further adsorption column being interposed between the adsorption column (24) for SO₂ and the adsorption column (27) for CO₂, and
**that** in front of and behind the adsorption column (27) for CO₂ a multiway valve (37; 44) is respectively provided, said multiway valves being switchable such that the gas flow can be interrupted by the one multiway valve (37) from the further adsorption column (25), which serves to separate the water vapor, to the CO₂ adsorption column (27) and the gas flow is passed by said multiway valve (37) via a reduction furnace (39) and the other multiway valve (44) to the IRMS measurement (29).

14. A device for determining the isotope ratios of the elements carbon (C), nitrogen (N) and sulfur (S) in one analytical run, the device comprising an elemental analyzer with a combustion furnace (1) for burning the sample, a successive column (24) fed with the combustion gases, which is filled with an SO₂ adsorbent, and a successive column (27) filled with a CO₂ adsorbent, and the combustion gases after the column (27) filled with the CO₂ adsorbent being passed via a thermal conductivity detector (30) to an IRMS measuring device (29),
**characterized in**
**that** for the determination of the isotope ratios of C, N, S and additionally also of H in one analytical run a further adsorption column (25) for separating the water vapor is provided, which is interposed between the adsorption column (24) for SO₂ and the adsorption column (27) for CO₂, and
**that** in front of and behind the adsorption column (27) for CO₂ a multiway valve (37; 44) is respectively provided, said multiway valves being switchable such that the gas flow can be interrupted by the one multiway valve (37) from the further adsorption column (25), which serves to separate the water vapor, to the CO₂ adsorption column (27) and the gas flow is supplied by the multiway valve (37) via the other multiway valve (44) to an equilibration unit (48) which is connected to the IRMS measuring device (29), together with H₂ gas of known isotope content which is provided by a unit (47).

15. The device according to claim 14, **characterized in that** the equilibration unit (48) is an H exchange reactor.

16. The device according to claim 13, **characterized in that** a reduction tube (39) positioned in the reduction furnace is filled with metals or metal combinations of suitable electronegativity.

17. The device according to claim 16, **characterized in that** the reduction tube (39) in the reduction furnace is filled with magnesium chips.

18. The device according to claim 16, **characterized in that** the reduction tube (39) in the reduction furnace is filled with manganese granulate.

19. The device according to claim 14, **characterized in that** after the equilibration unit (48) a device (49) is used for separating the water.

20. The device according to claim 13, **characterized in that** the combustion products at the output side of the reduction furnace (39) are passed through a drying tube (43).

21. The device according to claim 20, **characterized in that** the drying tube (43) is filled with phosphorus pentoxide.

22. The device according to any one of claims 13 to 21, **characterized in that** the adsorption columns (24, 25, 27) are designed for sample amounts above 10 mg.

23. The device according to any one of claims 13 to 22, **characterized in that** corresponding separation columns are used for adsorption/desorption.

24. The device according to any one of claims 13 to 23, **characterized in that** the interface between the elemental analysis and the isotope mass spectrometer analysis is designed according to the "continuous flow" principle.

## Revendications

1. Procédé destiné à la détermination des rapports isotopiques des éléments de carbone (C), de l'azote (N) et du soufre (S) avec un analyseur élémentaire en association avec un spectromètre de masse (EA-IRMS), les gaz de combustion issus des éléments C et S de N₂ étant séparés par au moins une colonne d'adsorption spécifique à l'élément et à commande thermique pour le SO₂ et ensuite par une colonne d'adsorption spécifique à l'élément et à commande thermique pour le CO₂ et étant ensuite amenés à une mesures IRMS,
**caractérisé en ce que**
pour la détermination des rapports isotopiques de C, N, S et en supplément également de H en une étape d'analyse, les gaz de combustion sont guidés par une autre colonne d'adsorption pour la séparation de la vapeur d'eau qui résulte de la combustion de l'hydrogène de l'échantillon qui est intercalée entre la colonne d'adsorption pour SO₂ et la colonne d'adsorption pour CO₂, et **en ce que** pour la détermination du rapport isotopique de H,
le courant de gaz est interrompu entre l'autre colonne d'adsorption qui sert à la séparation de la vapeur d'eau et la colonne d'adsorption CO₂ et que le courant de gaz est guidé soit par un four de réduction et est amené à la mesure IRMS
soit il est amené avec du gaz H₂ pour l'équilibre isotopique et l'hydrogène est amené à la mesure IRMS.

2. Procédé selon la revendication 1, **caractérisé en ce qu'**un tube de réduction se trouvant dans le four de réduction est remplis de copeaux de magnésium.

3. Procédé selon la revendication 1, **caractérisé en ce qu'**un tube de réduction se trouvant dans le four de réduction est remplis de granulé de manganèse.

4. Procédé selon la revendication 1, **caractérisé en ce qu'**un tube de réduction se trouvant dans le four de réduction est remplis de métaux ou de combinaisons de métaux d'électronégativité appropriée.

5. Procédé selon la revendication 1, **caractérisé en ce qu'**un équilibre isotopique catalysé Pt est effectué à 100° entre la vapeur d'eau et le gaz d'hydrogène de teneur isotopique connue dans un récipient et ensuite l'eau est séparée dans une unité de séchage.

6. Procédé selon la revendication 5, **caractérisé en ce que** la séparation de l'eau est effectuée dans un tube Nation^{®}.

7. Procédé selon l'une des revendications 1 à 4, **caractérisé en ce que** les produits de combustion sont soumis côté sortie du four de réduction à un séchage pour la protection contre la contamination par une humidité étrangère.

8. Procédé selon la revendication 7, **caractérisé en ce que** le séchage s'effectue dans un tube de séchage rempli de pentoxyde de phosphore.

9. Procédé selon la revendication 1, **caractérisé en ce que** la quantité de H₂O et d'hydrogène est déterminée par mesure de pression respectivement au moyen d'un détecteur de conductivité thermique (WLD).

10. Procédé selon la revendication 1, **caractérisé en ce que** les colonnes de séparation contiennent une quantité suffisante de matériau d'adsorption spécifique pour des quantités d'échantillon supérieure à 10 mg.

11. Procédé selon l'une des revendications 1 à 10, **caractérisé en ce que** l'interface entre l'analyse élémentaire et l'analytique de spectromètre de masse et de rapport isotopique est réalisée selon le principe du "continuous flow".

12. Procédé selon l'une des revendications 1 à 11, **caractérisé en ce que** pour la détermination des rapports isotopiques de C, H, N et S des pesées nettes allant jusqu'à 100 mg sont utilisés.

13. Dispositif pour la détermination des rapports isotopiques des éléments de carbone (C), de l'azote (N) et du soufre (S) en une étapes d'analyse, dispositif qui présente un analyseur élémentaire avec un four de combustion (1) pour la combustion de l'échantillon avec une colonne suivante (24) à laquelle sont amenés les gaz de combustion, et est remplie d'agent d'adsorption SO₂ et d'une colonne suivante (27) qui est remplie d'agent d'adsorption CO₂ et les gaz de combustion étant amenés après la colonne (27) remplie d'agent adsorption de CO₂ par un détecteur de conductibilité thermique (30) à un dispositif de mesure IRMS (29),
**caractérisé en ce que**
pour la détermination des rapports isotopiques de C, N, S et en supplément de H en une étape d'analyse une autre colonne d'adsorption (25) est prévue pour la séparation de la vapeur d'eau qui est intercalée entre la colonne d'adsorption (24) pour SO₂ et la colonne d'adsorption (27) pour le CO₂ et
devant et derrière la colonne d'adsorption (27) pour le CO₂, il est prévu respectivement une soupape multivoie (37; 44) qui peut être commutée de sorte que le courant de gaz est interrompu par la soupape multivoie (37) entre l'autre colonne d'adsorption (25) servant à la séparation de vapeur d'eau et la colonne d'adsorption CO₂ (27), et le courant de gaz est amené par cette soupape multivoie (37) par un four de réduction (39) et l'autre soupape multivoie (44) à la mesure IRMS (29).

14. Dispositif pour la détermination des rapports isotopiques des éléments de carbone (C), de l'azote (N) et du soufre (S) en une étapes d'analyse, dispositif qui présente un analyseur élémentaire avec un four de combustion (1) pour la combustion de l'échantillon avec une colonne suivante (24) à laquelle sont amenés les gaz de combustion, et est remplie d'agent d'adsorption SO₂ et d'une colonne suivante (27) qui est remplie d'agent d'adsorption CO₂ et les gaz de combustion étant amenés après la colonne (27) remplie d'agent adsorption de CO₂ par un détecteur de conductibilité thermique (30) à un dispositif de mesure IRMS (29),
**caractérisé en ce que**
pour la détermination des rapports isotopiques de C, N, S et en supplément de H en une étape d'analyse une autre colonne d'adsorption (25) est prévue pour la séparation de la vapeur d'eau qui est intercalée entre la colonne d'adsorption (24) pour SO₂ et la colonne d'adsorption (27) pour le CO₂ et
devant et derrière la colonne d'adsorption (27) pour le CO₂, il est prévu respectivement une soupape multivoie (37; 44) qui peut être commutée de sorte que le courant de gaz est interrompu par la soupape multivoie (37) entre l'autre colonne d'adsorption (25) servant à la séparation de vapeur d'eau et la colonne d'adsorption CO₂ (27), et le courant de gaz est amené par cette soupape multivoie (37) par l'autre soupape multivoie (44) à une unité d'équilibre (48) qui est reliée au dispositif de mesure IRMS (29), ensemble avec le gaz H₂ de teneur isotopique connue, gaz H₂ qui est mis à disposition par une unité (47).

15. Dispositif selon la revendication 14, **caractérisé en ce que** l'unité d'équilibre (48) est un réacteur d'échange H.

16. Dispositif selon la revendication 13, **caractérisé en ce qu'**un tube de réduction (39) se trouvant dans le four de réduction est remplis de métaux ou de combinaisons de métaux d'électronégativité appropriée.

17. Dispositif selon la revendication 16, **caractérisé en ce que** le tube de réduction (39) se trouvant dans le four de réduction est rempli de copeaux de magnésium.

18. Dispositif selon la revendication 16, **caractérisé en ce que** le tube de réduction (39) se trouvant dans le four de réduction est rempli de granulé de manganèse.

19. Dispositif selon la revendication 14, **caractérisé en ce que** l'unité d'équilibre (48) à un dispositif (49) est utilisée pour la séparation de l'eau.

20. Dispositif selon la revendication 13, **caractérisé en ce que** les produits de combustion sont guidés côté sortie du tube de réduction (39) par un tube de séchage (43).

21. Dispositif selon la revendication 20, **caractérisé en ce que** le tube de séchage (43) est rempli de pentoxyde de phosphore.

22. Dispositif selon l'une des revendications 13 à 21, **caractérisé en ce que** les colonnes d'adsorption (24, 25, 27) sont conçues pour des quantités d'échantillon supérieures à 10 mg.

23. Dispositif selon l'une des revendications 13 à 22, **caractérisé en ce que** des colonnes de séparation sont utilisées pour l'adsorption/désorption.

24. Dispositif selon l'une des revendications 13 à 23, **caractérisé en ce que** l'interface entre l'analyse élémentaire et l'analytique de spectromètre de masse et de rapport isotopique est réalisée selon le principe du "continuous flow".
